# EUROPEAN PATENT APPLICATION

(11) **EP 4 471 425 A1**
(43) Date of publication of application: **04.12.2024**
(21) Application number: 23780285.5
(22) Date of filing: 27.03.2023
(51) Int. Cl.: G01N 33/543, G01N 33/545

(54) **LATEX PARTICLE DISPERSION LIQUID**

(30) Priority: 28.03.2022 JP 2022051534
(71) Applicant: Sekisui Medical Co., Ltd., Tokyo 103-0027 (JP)
(72) Inventor: FUJIMURA, Kengo, Tokyo 103-0027 (JP); BANBA, Yoshimasa, Tokyo 103-0027 (JP); YASUHARA, Kaori, Tokyo 103-0027 (JP); HORIUCHI, Ryosuke, Tokyo 103-0027 (JP); KAGAYA, Hiroshi, Tokyo 103-0027 (JP); KORA, Misato, Tokyo 103-0027 (JP); ISHIHARA, Yuka, Tokyo 103-0027 (JP); MASUDA, Yuuta, Tokyo 103-0027 (JP)
(74) Representative: Hoffmann Eitle
(86) International application number: PCT/JP2023/012073
(87) International publication number: WO 2023/190275

(57) **Abstract**

An objective of the present invention is to provide a technique for increasing sensitivity of latex immunoagglutination reagents while inhibiting sedimentation of particles in a latex particle dispersion liquid. In particular, it is to provide the latex particle dispersion liquid which can be applied to the latex immunoagglutination reagents with adequate sensitivity while inhibiting occurrence of agglutination over time in the latex particle dispersion liquid having a large particle diameter. That is, it is to provide the latex particle dispersion liquid using an aqueous medium as a dispersion medium, and including one or more compounds selected from the group consisting of polyacrylic acid, sodium polyacrylate, polyethylene oxide, and sodium poly-γ-glutamate. It is also to provide immunoagglutination reagents to which the latex particle dispersion liquid is applied.

## Description

### [Technical Field]

The present invention relates to a latex particle dispersion liquid. It also relates to a method for detecting a target substance in a specimen by an immunoagglutination method using the dispersion liquid, and a detection kit. It further relates to a method for inhibiting sedimentation of latex particles.

### [Background Art]

The latex immunoagglutination method (also referred to as Latex turbidimetrical immunoassay: LTIA) is a well-known method for qualitative or quantitative measurement of a target substance in a biological sample. The above method is known as a method for optically detecting a degree of agglutination of latex particles resulting from an immune reaction between an antigen or antibody carried on surfaces of the latex particles and an antibody or antigen that is the target substance in the biological sample. The latex particles carrying the antigen or antibody are dispersed in an aqueous medium such as a buffer solution. The aqueous medium containing the latex particles is hereinafter referred to as the latex particle dispersion liquid. The latex particle dispersion liquid can be used as a measuring reagent according to the principle of the latex immunoagglutination method and is hereinafter referred to as a latex reagent.

Applying the measuring reagent to an automated analyzer allows a large number of samples to be measured easily and quickly. The reagent is stored in a container and in a reagent storage of the automated analyzer. From a viewpoint of usability, it is desirable that the composition of reagent remains uniform over a long period of time (good on-board stability) in a state in which the container is left to stand in the reagent storage.

However, in the reagent of the latex immunoagglutination method, when a particle diameter of the latex particles is increased to improve sensitivity, the latex particles sediment over time after the reagent is filled into the container, and the latex reagent has become uneven. When a sample is measured with such an uneven latex reagent, the problem of being unable to obtain accurate readings arises. Therefore, in order to perform accurate measurements using the reagents containing latex particles having large particle diameters, it was necessary to redisperse the latex particles in the aqueous medium by overturn mixing the reagent container or stirring the reagent prior to measurement.

On the other hand, as techniques for inhibiting sedimentation of microparticles in a reagent or for improving dispersibility of the microparticles, the following are known. In Patent Literature 1, as a method for inhibiting sedimentation of microparticles to which reactive substances such as antigens and antibodies are bound, disclosed is a method in which at least one selected from the group consisting of polyanion and a salt thereof, dextran, cyclodextrin, polyethylene glycol, and glycerol is made to coexist in a dispersion liquid of the microparticles. However, the sedimentation inhibiting effect was confirmed only for gold colloidal particles of 5 to 100 nm, and the sedimentation inhibiting effect for latex particles was unknown.

In Patent Literature 2, described is a reagent containing a specific amount of sugar such as sucrose, glucose, trehalose, and the like, in a reagent for latex turbidimetrical immunoassay composed of a suspension of latex particles carrying an antigen or antibody, to suppress particle sedimentation while maintaining high reagent sensitivity, in the reagent for the latex turbidimetrical immunoassay. This is the technique that attempts to improve dispersibility by increasing the specific gravity of the suspension by incorporating a specific amount of the sugars into the particle suspension.

With respect to these sugars, the present inventors have studied the sedimentation inhibiting effects of the latex particles and the effects on the measurement sensitivity of the reagent. However, a new problem has arisen because the measurement sensitivity of the reagent has decreased, although they have shown a sedimentation inhibiting effect (see Comparative Examples 2, 3 in the Examples described later).

In summary, there has been no technique to date that does not have undesirable effects on reagent performance such as reduced sensitivity of latex immunoagglutination reagents, while at the same time inhibiting sedimentation of particles in the latex particle dispersion liquid.

### [Citation List]

### [Patent Literature]

[Patent Literature 1]
   Japanese Patent Laid-Open No. 2007-114129
[Patent Literature 2]
   Japanese Patent Laid-Open No. 11-051938

### [Summary of Invention]

### [Technical Problem]

An objective of the present invention is to provide a technique for increasing sensitivity of latex immunoagglutination reagents while improving dispersibility of particles in a latex particle dispersion liquid and inhibiting particle sedimentation. In particular, the objective is to inhibit sedimentation and/or agglutination of latex particles in a dispersion liquid containing latex particles having large particle diameters.

### [Solution to Problem]

To perform accurate and sensitive measurements with the latex immunoagglutination reagents, it is necessary to have a technique that disperses the latex particles uniformly and stably in the latex reagent.

In order to solve the above problem, the present inventors have made intensive studies and found that coexistence of one or more compounds selected from the group consisting of polyacrylic acid, sodium polyacrylate, polyethylene oxide (hereinafter simply referred to as PEO), and sodium poly-γ-glutamate in the latex particle dispersion liquid carrying a substance capable of binding to a target substance inhibits the sedimentation of the latex particles for a long time, and that the measurement sensitivity is maintained or improved as compared with reagents to which those components are not added, when applied to the immunoagglutination reagents, and the present invention has been completed. Namely, the present invention includes the following.
<1> A latex particle dispersion liquid including an aqueous medium as a dispersion medium, wherein the latex particle dispersion liquid includes one or more compounds selected from the group consisting of polyacrylic acid, sodium polyacrylate, polyethylene oxide, and sodium poly-**γ-**glutamate.
<2> The latex particle dispersion liquid according to <1>, wherein the aqueous medium includes water.
<3> The latex particle dispersion liquid according to <1> or <2>, wherein the latex particles are polymer particles derived from a monomer component containing a monomer having a styrene or vinylnaphthalene structure.
<4> The latex particle dispersion liquid according to any one of <1> to <3>, wherein the latex particles are latex particles carrying a substance capable of binding to a target substance.
<5> The latex particle dispersion liquid according to <4>, wherein the substance capable of binding to the target substance is an antigen or antibody to the target substance.
<6> The latex particle dispersion liquid according to any one of <1> to <5>, wherein the latex particle dispersion liquid is for use as an immunoagglutination reagent.
<7> The latex particle dispersion liquid according to any one of <1> to <6>, wherein an average particle diameter of the latex particles is 50 to 1,000 nm.
<8> The latex particle dispersion liquid according to any one of <1> to<7>, wherein a content of the latex particles is 0.01 to 0.10% by mass with respect to a total amount of the latex particle dispersion liquid.
<9> A reagent kit for use in detecting a target substance in a specimen by an immunoagglutination method, the reagent kit including the latex particle dispersion liquid according to any one of <1> to <8>.
<10> A method for detecting a target substance in a specimen by an immunoagglutination method, the method including a step of bringing the specimen into contact with the latex particle dispersion liquid according to any one of <1> to <8>.
<11> A method for inhibiting sedimentation of latex particles carrying a substance capable of binding to a target substance in an immunoagglutination method,
   the method including a step of bringing the latex particles into contact with one or more compounds selected from the group consisting of polyacrylic acid, sodium polyacrylate, polyethylene oxide, and sodium poly-γ-glutamate in an aqueous medium.

### [Advantageous Effect of Invention]

According to the latex particle dispersion liquid of the present invention, since sedimentation and/or agglutination of latex particles is inhibited, when applied to latex immunoagglutination reagents, the latex reagents can be used for measurement without redispersing the latex particles by overturn mixing a reagent container, stirring the reagents, and the like. In addition, the on-board stability of the immunoagglutination reagents using the latex particles can be improved.

### [Brief Description of Drawings]

[Figure 1] Figure 1 shows graphs of absorbance of TARC calibration substances at each concentration when measured (Comparative Examples 1 to 3, Examples 1 to 9).
[Figure 2] Figure 2 shows graphs of the absorbance of the TARC calibration substances at each concentration when measured (Examples 10 to 21).

### [Description of Embodiment]

### (Latex Particle Dispersion Liquid)

A latex particle dispersion liquid of the present invention in which an aqueous medium is a dispersion medium includes one or more compounds selected from the group consisting of polyacrylic acid, sodium polyacrylate, PEO, and sodium poly-**γ**-glutamate. Coexistence of these compounds with latex particles has made it possible to improve dispersibility of the latex particles in the aqueous media.

As the polyacrylic acid used in the latex particle dispersion liquid of the present invention, those having an average molecular weight of 5,000 to 1,000,000 are preferable, and commercially available products include polyacrylic acid 5,000, polyacrylic acid 25,000, polyacrylic acid 250,000, polyacrylic acid 1,000,000 (produced by FUJIFILM Wako Pure Chemical Corporation), etc.

As the sodium polyacrylate used in the latex particle dispersion liquid of the present invention, those having a polymerization degree of 2,700 to 70,000 are preferable, and commercially available products include sodium polyacrylate 2,700 to 7,000, sodium polyacrylate 22,000 to 70,000 (produced by FUJIFILM Wako Pure Chemical Corporation), etc.

As the polyethylene oxide (PEO) used in the latex particle dispersion liquid of the present invention, those having a molecular weight of 100,000 to 2,200,000 are preferable, and PEO having such molecular weights include PEO indicated by the grade symbol PEO-1 (molecular weight of 150,000 to 400,000), PEO indicated by the grade symbol PEO-2 (molecular weight of 400,000 to 600,000), PEO indicated by the grade symbol PEO-3 (molecular weight of 600,000 to 1,100,000), PEO indicated by the grade symbol PEO-4 (molecular weight of 1,100,000 to 1,500,000), and PEO indicated by the grade symbol PEO-8 (molecular weight of 1,700,000 to 2,200,000) (produced by SUMITOMO SEIKA CHEMICALS CO., LTD.), and other PEO (molecular weight of 100,000, 200,000, 400,000, 600,000, 1,000,000, produced by Sigma-Aldrich), etc. (The above molecular weights for Sigma-Aldrich's PEO indicate a viscosity-averaged molecular weight).

As the sodium poly-**γ**-glutamate used in the latex particle dispersion liquid of the present invention, those having an average molecular weight of 400,000 or more are preferable, and commercially available products include sodium poly-**γ**-glutamate (produced by FUJIFILM Wako Pure Chemical Corporation).

The above compounds can be used in one type or in combination of two or more types.

A content of the compound described above in the latex particle dispersion liquid is preferably 0.002 to 0.50% by mass, more preferably 0.003 to 0.30% by mass, and most preferably 0.005 to 0.20% by mass. This is because that if it is more than 0.50% by mass (upper limit), the latex particles may agglutinate or viscosity of a reagent may increase, resulting in cases of decreased measurement accuracy, while if it is less than 0.002% by mass (lower limit), there is inferior in sedimentation inhibiting effect of the latex particles in the liquid. A range of 0.002% by mass (lower limit) to 0.50% by mass (upper limit) is suitable because it prevents the latex particles from agglutinating and increasing the viscosity of the reagent, which are undesirable effects on accuracy of the reagent, while maintaining the sedimentation inhibiting effect of the latex particles.

pH of the latex particle dispersion liquid of the present invention is preferably pH 4.0 to pH 10.0, more preferably pH 5.0 to pH 9.0, and most preferably pH 6.0 to pH 8.0.

The pH can be adjusted using pH adjusting reagents well known to those skilled in the art, such as sodium hydroxide and hydrochloric acid.

The latex particle dispersion liquid of the present invention can be used as an immunoagglutination reagent and can also be combined with other reagent configurations into a kit. The immunoagglutination reagents typically include in vitro diagnostic reagents. The reagent kit configurations will be described later.

### (Aqueous Medium)

An aqueous medium used in the latex particle dispersion liquid of the present invention is any medium capable of dispersing the latex particles, and among these, those containing water are preferable. There is no limitation on a content of water as long as it is capable of dispersing the latex particles, and ranges of the content of water include, e.g., 40 to 100% by mass, 60 to 100% by mass, 80 to 100% by mass, 90 to 100% by mass, etc. with respect to the total amount of the aqueous medium.

The aqueous medium can also be a buffer solution such as a Good's buffer solution, a Tris buffer solution, a phosphate buffer solution, a glycine buffer solution, an ammonia buffer solution, a citrate buffer solution, an acetate buffer solution, and a succinate buffer solution.

In addition to the water and buffer solution described above, the aqueous medium may contain lower alcohols such as methanol, ethanol, and isopropanol, and other organic solvents that mix with water such as dimethyl sulfoxide and dimethyl formamide.

A content of the aqueous medium includes, e.g., 50 to 99.9% by mass and 70 to 99.9% by mass with respect to the total amount of the latex particle dispersion liquid.

### (Latex Particles)

In the latex particle dispersion liquid of the present invention, the latex particles are not limited as long as they are latex particles commonly used as reagents for immunoassay. The latex particles can be obtained by polymerization or copolymerization of various monomers. Herein, the monomers include,
polymerizable unsaturated aromatics: e.g., polymerizable monomers having a phenyl group, such as styrene, **α**-methylstyrene, o-methylstyrene, p-methylstyrene, p-chlorostyrene, 4-vinylbenzoate, divinylbenzene, and vinyltoluene; polymerizable monomers having a phenyl group and a sulfonic acid salt, such as styrenesulfonate, divinylbenzenesulfonate, o-methylstyrenesulfonate, and p-methylstyrenesulfonate; and polymerizable monomers having a naphthyl group, such as 1-vinylnaphthalene, 2-vinylnaphthalene, **α**-naphthyl (meth)acrylate, and **β**-naphthyl (meth)acrylate:
polymerizable unsaturated carboxylic acids: e.g., such as (meth)acrylic acid, itaconic acid, maleic acid, and fumaric acid:
polymerizable unsaturated carboxylic esters: e.g., such as methyl (meth)acrylate, ethyl (meth)acrylate, (meth)acrylic acid-n-butyl, (meth)acrylic acid-2-hydroxyethyl, glycidyl (meth)acrylate, ethylene glycol-di-(meth)acrylic esters, and tribromophenyl (meth)acrylate:
unsaturated carboxylic acid amides, such as (meth)acrylonitrile, (meth)acrolein, (meth)acrylamide, N-methylol-(meth)acrylamide, methylenebis(meth)acrylamide, butadiene, isoprene, acetic acid vinyl, vinylpyridine, N-vinylpyrrolidone, vinyl chloride, vinylidene chloride, and vinyl bromide:
polymerizable unsaturated nitriles:
vinyl halides: and
conjugated dienes.

Among these, polymer particles derived from the monomer component containing the monomer having the styrene or vinylnaphthalene structure are preferable.

These monomers are appropriately selected according to the required surface properties, specific gravity, etc., and can be used in one type alone or in a mixture of two or more types.

Synthetic polymers constituting the latex particles of the present invention are not limited, but include, e.g., polystyrene, a styrene-styrenesulfonate copolymer, a methacrylic acid polymer, an acrylic acid polymer, an itaconic acid polymer, a styrene-hydrophilic carboxy monomer copolymer, such as a styrene-(meth)acrylic acid copolymer, a styrene-acrylic acid copolymer, and a styrene-itaconic acid copolymer, etc. Among them, the styrene-methacrylic acid copolymer, the styrene-itaconic acid copolymer, the styrene, and the styrene-styrenesulfonate copolymers are preferable.

It is preferable that a content of the latex particles is 0.01 to 0.10% by mass with respect to the total amount of the latex particle dispersion liquid.

An average particle diameter of the latex particles in the present invention should be a size sufficient to obtain sensitivity differences in a measurement object and a measurement range, and e.g., 50 nm to 1,000 nm is preferable. Although larger average particle diameters of the latex particles increase sensitivity, the particles tend to sediment easily, making it difficult to apply them to measuring reagents, but the latex particle dispersion liquid of the present invention has a property of being resistant to sedimentation even when the particle diameter is large. Therefore, when enjoying the maximum effect of the present invention, the larger the average particle diameter of the latex particles, the more preferable, and it is preferably 100 nm or more, more preferably 200 nm or more, and even more preferably 300 nm or more.

It is also possible to further expand the measurable concentration range by using latex particles with two or more average particle diameters, large and small.

The average particle diameter of the latex particles can be analyzed by a laser diffraction and scattering (LS) method, Coulter principle, a dynamic light scattering photon correlation method, an electron microscopy, etc. In the present description, the average particle diameter means a volume average particle diameter.

### (Substance Capable of Binding to Target Substance)

In the present invention, substances capable of binding to target substances include proteins, peptides, amino acids, lipids, carbohydrates, nucleic acids, haptens, etc., and there is no limitation as to high or low of the molecular weight, and natural or synthetic origin, but include antibodies or antigens that can be used in an immunoassay method utilizing immunoreaction.

The antibodies can be either polyclonal or monoclonal antibodies. They are more preferably monoclonal antibodies.

As the antibodies used in the present invention, it is also possible to use functional fragments of antibodies with antigen-antibody reaction activity in addition to whole antibody molecules. In addition to antibodies obtained by an immunization procedure in common animals (mice, goats, sheep, etc.), antibodies (chimeric antibodies, humanized antibodies, fully humanized antibodies, or the like) in which the immunogen (substance to be measured) is modified to the amino acid sequence of an animal species other than that to be immunized by genetic recombination technology, etc. are also acceptable. The functional fragments of antibodies include, F(ab')₂, Fab', which are fragments with the antigen-antibody reaction activity, and single-chain antibodies (scFv), VHH (variable domain of heavy chain of heavy chain antibodies) antibodies, IgNAR (new antigen receptor) antibodies, etc. The functional fragments of these antibodies can be produced by treating the antibodies obtained as described above with proteolytic enzymes (e.g., pepsin, papain, or the like).

In the present invention, a substance capable of binding to a target substance to be carried on the latex particles can be immobilized and carried on the latex particles by known methods such as a physical adsorption method, a chemical binding method, and a combination of these methods.

In a case by the physical adsorption method, it can be done according to a known method, by mixing and bringing the substance capable of binding to the target substance into contact with the latex particles in a solution such as a buffer solution, or by bringing the substance capable of binding to the target substance dissolved in a buffer solution or the like into contact with a carrier, and the like.

In a case performed by the chemical binding method, according to a known method described in, "Immunoassay for Clinical Tests - Techniques and Applications -, Clinical Pathology Extra Edition No. 53", edited by the Japanese Association of Clinical Pathology, and published by Clinical Pathology Publishing Association, 1983, and "New Biochemistry Experimental Course 1, Protein IV", edited by Japanese Biochemical Society, and published by Tokyo Chemistry Coterie, 1991, etc., the method can be performed by mixing and bringing a substance capable of binding to the target substance, the latex particles, and a bivalent cross-linking reagent such as glutaraldehyde, carbodiimide, imido ester, maleimide, or the like, into contact, to react an amino group, a carboxyl group, a thiol group, an aldehyde group, or a hydroxyl group of each of the substance binding to the target substance, and the latex particles with the above bivalent cross-linking reagent.

If some treatment is required to inhibit spontaneous agglutination of the latex particles and non-specific reactions, surfaces of the latex particles may be treated by known methods of contacting and coating with: proteins such as bovine serum albumin (BSA), casein, gelatin, and egg white albumin or a salt thereof; surfactants; nonfat milk powder; or the like; to perform blocking (masking) treatment of carriers.

### (Reagent Kit)

A reagent kit for use in detecting a target substance in a specimen by the immunoagglutination method of the present invention includes at least the latex particle dispersion liquid of the present invention described above. Typically, a kit configuration includes a two-reagent system configuration provided with a particle dispersion liquid (2nd reagent) containing particles carrying a substance that binds to the target substance and a 1st reagent containing a buffer solution. The buffer solution includes a Good's buffer, a Tris buffer, a phosphate buffer, a glycine buffer, an ammonia buffer, a citrate buffer, an acetate buffer, a succinate buffer, etc. In addition to the buffer solution, the 1st reagent may also contain a blocking agent or an agglutination promoter. In addition to the 1st and 2nd reagents, the kit configuration also includes a sample diluent, a sample extraction solution, instructions for use, and sample collection tools (a collection pipette, a syringe, a cotton swab, a filtration filter, etc.).

### (Method for Detecting Target Substance)

A method for detecting a target substance in a specimen by the immunoagglutination method of the present invention is performed by bringing the specimen into contact with the latex particle dispersion liquid described above, and the above immunoagglutination method is also referred to as the latex immunoagglutination method (LTIA method). The method for measuring the target substance by the LTIA method can be broadly classified into two categories.

The first is a method in which latex particles carrying a substance capable of binding to a target substance are reacted with the target substance to form a sandwich-type immune complex, and the target substance is measured by the degree of agglutination of the latex particles associated with the formation of the immune complex.

The second is a method in which proteins, etc. immobilizing a plurality of target substances or their analogs (including their fragments) are added to a reagent and compete with the target substance in the sample to inhibit the formation of an immune complex between the target substance contained in the reagent and latex particles carrying a substance capable of binding the target substance, and the target substance (antigen) is measured by the degree of inhibition of agglutination of the latex particles associated with the inhibition of the formation of the immune complex.

As the target substance and the substance capable of binding to the target substance, any substance can be selected depending on the purpose. For example, when the target substance is an antigen, antibodies such as polyclonal antibodies and monoclonal antibodies (including recombinant antibodies and functional fragments of each antibody) can be selected as substances capable of binding to the target substance. When the target substance is an antibody, antigens such as natural and recombinant antigens can be selected as substances capable of binding to the target substance. The present invention can be used for any of the above methods, and specifically, the following steps are exemplary.
(1) A step of mixing the target substance in the specimen with the buffer solution or the like.
(2) After the step (1), a step in which a latex particle dispersion liquid carrying a substance capable of binding to the target substance is added to the mixed solution, and brought it into contact with the specimen.
(3) After the step (2), a step of optically detecting the degree of agglutination of the latex particles in the liquid.

Here, the step (3) means "a step of measuring an agglutination reaction of the target substance and the latex particles without going through washing and separation steps during or after the step (2)".

In the present invention, as methods for optically observing the degree of agglutination of the latex particles, examples include methods for measuring scattered light intensity, absorbance, or transmitted light intensity by optical instruments (an end point method, a rate method, etc.). By comparing the measured values of absorbance, etc. obtained by measuring the sample with the measured values of absorbance, etc. obtained by measuring a standard substance (sample with a known concentration of the substance to be measured), the concentration of the substance to be measured contained in the sample (a quantitative value) can be calculated. The measurement of the absorbance, etc. of transmitted or scattered light, or the like can be a single-wavelength measurement or a two-wavelength measurement (a difference or ratio between two wavelengths). The measured wavelength is usually selected from 500 nm to 900 nm.

The measurement of the target substance in the specimen of the present invention may be performed by a manual method or by using a device of a measuring instrument or the like. The measuring instrument may be a general-purpose automatic analyzer or a dedicated measuring instrument (specialized machine).

### (Sedimentation Inhibiting Method)

A method for inhibiting sedimentation of latex particles carrying a substance capable of binding to a target substance in the immunoagglutination method of the present invention includes a step of bringing the latex particles into contact with one or more compounds selected from the group consisting of polyacrylic acid, sodium polyacrylate, PEO, and sodium poly-γ-glutamate in an aqueous medium. It is considered that bringing the latex particles into contact with the compound in an aqueous medium inhibits sedimentation of the latex particles and improves dispersibility, although the reason is not clear.

In the present application, "sedimentation of latex particles" includes both cases where the particles sediment by themselves and cases where the particles increase in size and sediment by adsorption or agglutination with each other. Furthermore, in the present application, "inhibiting sedimentation of latex particles" includes the case where the particles are inhibited from sedimentation as they are, the case where the particles are inhibited from sedimentation by increasing their size as a result of inhibiting adsorption or agglutination of the particles, and the case where both of them are inhibited.

In the present description, the presence or absence of a sedimentation inhibiting effect of latex particles can be evaluated, for example, as shown in the examples described later, such that when the additive to be tested is added to the latex particle dispersion liquid and a content ratio of the latex particles in a liquid surface of the dispersion liquid is higher than that in the case without the additive, the sedimentation inhibiting effect of the additive is present.

### (Specimen)

Specimens containing a target substance in the present invention include samples obtained from living organisms such as, e.g., human or animal blood, serum, plasma, culture supernatant, urine, spinal fluid, saliva, sweat, ascites fluid, and cell or tissue extracts.

In addition, specimens in the present invention include samples themselves obtained from living organisms as well as samples that have undergone pretreatments such as dilution, purification, extraction, and filtration treatments. Blood specimens include whole blood, red blood cells, plasma, serum, etc., and also include plasma specimens collected through a blood collection tube to which an anticoagulant has been added.

### (Target Substance)

Target substances in the present invention include proteins, peptides, amino acids, lipids, carbohydrates, nucleic acids, haptens, etc., but there is no limitation as long as the substances are theoretically measurable. Examples include CRP (C-reactive protein), Lp(a), MMP3 (matrix metalloproteinase 3), antiphospholipid antibodies, type IV collagen, PSA, BNP (brain natriuretic peptide), insulin, albumin, cystatin C, RF (rheumatoid factor), KL -6, procalcitonin, FDP, D-dimer, SF (soluble fibrin), TAT (thrombin-antithrombin III complex), PAI-1, phenytoin, phenobarbital, carbamazepine, valproic acid, theophylline, TARC (Thymus and activation-regulated chemokine), sIL-2R (soluble interleukin-2 receptor), etc.

Hereinafter, the present invention will be described in detail with reference to Examples, but the present invention is not limited to the following Examples.

### Examples

### [Test Example 1: Evaluation of Latex Particle Sedimentation]

Latex particle dispersion liquid (2nd reagent of LTIA measuring reagent) was formulated with various additives and stored, and the sedimentation of latex particles in a solution was evaluated.

### [Comparable Example 1: Without Additive]

The test and evaluation methods are as follows. The composition of the reagents is shown below.

### (1) Preparation of 2nd Reagent of LTIA Measuring Reagent 5 mM MOPS-NaOH (pH 7.0)

### Anti-human TARC monoclonal antibody sensitized latex (2 types)

In addition, the anti-human TARC monoclonal antibody was obtained using commercially available TARC antigen (*) by a method well known to those skilled in the art. Further, a combination of the monoclonal antibody capable of a sandwich-type measurement to the TARC antigen was selected by a method well known to those skilled in the art. The anti-human TARC monoclonal antibody sensitized latex was prepared with reference to the method described in Japanese Patent Laid-Open No. 2017-181377. That is, to a 1.0% latex solution with a mean particle diameter of 0.3 µm (5 mM Tris buffer (hereinafter, referred to as Tris-HCl or simply Tris)(pH 8.5)), an anti-TARC antibody solution, which was diluted to 0.36 mg/mL with an equal amount of 5 mM Tris-HCl (pH 8.5) was added and stirred at 4°C for 2 hours. Then, 5 mM Tris-HCl (pH 8.5) containing an equal amount of 0.5% BSA was added and stirred at 4°C for 1 hour to prepare the anti-TARC antibody sensitized latex particle solution. The anti-TARC antibody sensitized latex particle solution was diluted with 5 mM MOPS-NaOH (pH 7.0) to give an absorbance at 600 nm of approximately 5.5 OD, and used as the 2nd reagent of the LTIA measuring reagent.

(*) Commercially Available Antigens:
CCL 17, thymus and activation regulated chemokine (Shenandoah Biotechnology, Inc.), CCL 17/TARC, Human (LifeSpan Biosscience, Inc.), Human TARC (CCL17) (Abeomics, Inc.), etc.

### (2) Storage Conditions

After dispensing 5 mL of the above 2nd reagent to each of two glass test tubes, the tubes were left to stand at 2 to 8°C for 17 days.

### (3) Evaluation Method

One of the two glass test tubes was taken from a state of overturn mixed and the other from a state of left to stand, 100 µL was taken from a liquid surface to mix with 900 µL of 5 mM MOPS-NaOH (pH 7.0) (10-fold dilution), and the absorbances at 600 nm and 800 nm were measured. A ratio of the absorbance at 600 nm to that at 800 nm (600/800 ratio) was calculated as an index of the dispersibility of the particles in the solution. The ratio of absorbance at 600 nm between (without overturn mixing) and (after overturn mixing) was calculated as a content ratio (%) of latex particles on the liquid surface. The measurement results are shown in Table 2. For the absorbances at 600 nm and 800 nm, the measured values were multiplied by 10 and listed in Table 2 as the absorbances of the undiluted solution.

### [Comparative Examples 2 to 3: Formulated with Sucrose]

Measurements were performed in the same manner, except that sucrose (final concentration was listed in Table 1) was added to the 2nd reagent of the LTIA measuring reagents shown in Comparative Example 1. The measurement results are shown in Table 2.

### [Examples 1 to 21: Formulated with Additive]

The measurements were performed in the same manner, except that each additive (name and final concentration were listed in Table 1) was added to the 2nd reagent of the LTIA measuring reagents shown in Comparative Example 1. The measurement results are shown in Table 2. However, Mv indicates the molecular weight in the component names of Examples 16 to 20.

**[Table 1]**

| | | Additive | Concentration |
|---|---|---|---|
| Comparative Examples | 2 | Sucrose | 10% |
| | 3 | Sucrose | 5% |
| Examples | 1 | Polyacrylic acid 5,000 | 0.01% |
| | 2 | Polyacrylic acid 25,000 | 0.005% |
| | 3 | Polyacrylic acid 25,000 | 0.01% |
| | 4 | Polyacrylic acid 25,000 | 0.02% |
| | 5 | Polyacrylic acid 25,000 | 0.05% |
| | 6 | Polyacrylic acid 250,000 | 0.01% |
| | 7 | Polyacrylic acid 1,000,000 | 0.01% |
| | 8 | Sodium Polyacrylate 2,700 to 7,000 | 0.01% |
| | 9 | Sodium Polyacrylate 22,000 to 70,000 | 0.01% |
| | 10 | PEO-3 | 0.01% |
| | 11 | PEO-3 | 0.05% |
| | 12 | PEO-3 | 0.1% |
| | 13 | PEO-3 | 0.2% |
| | 14 | PEO-1 | 0.1% |
| | 15 | PEO-8 | 0.1% |
| | 16 | PEO Mv100,000 | 0.1% |
| | 17 | PEO Mv200,000 | 0.1% |
| | 18 | PEO Mv400,000 | 0.1% |
| | 19 | PEO Mv600,000 | 0.1% |
| | 20 | PEO Mv1,000,000 | 0.1% |
| | 21 | Sodium Poly-γ-Glutamate | 0.01% |

**[Table 2]**

| | Comparative Examples | 1 | 2 | 3 |
|---|---|---|---|---|
| With Overturn Mixing | 600 nm (OD) | 5.637 | 5.534 | 5.707 |
| | 800 nm (OD) | 2.548 | 2.499 | 2.575 |
| | 600/800 Ratio | 2.21 | 2.21 | 2.22 |
| Without Overturn Mixing | 600 nm | 3.076 | 4.464 | 5.114 |
| | 800 nm | 1.384 | 2.009 | 2.307 |
| | 600/800 Ratio | 2.22 | 2.22 | 2.22 |
| Without Overturn Mixing / After Overturn Mixing | Content Ratio (%) | 54.6% | 80.7% | 89.6% |

| | Examples | 1 | 2 | 3 | 4 | 5 | 6 | 7 | 8 | 9 |
|---|---|---|---|---|---|---|---|---|---|---|
| With Overturn Mixing | 600 nm (OD) | 5.786 | 5.748 | 5.885 | 5.271 | 5.607 | 5.745 | 5.137 | 5.690 | 5.611 |
| | 800 nm (OD) | 2.619 | 2.598 | 2.658 | 2.381 | 2.528 | 2.591 | 2.321 | 2.566 | 2.534 |
| | 600/800 Ratio | 2.21 | 2.21 | 2.21 | 2.21 | 2.22 | 2.22 | 2.21 | 2.22 | 2.21 |
| Without Overturn Mixing | 600 nm (OD) | 4.355 | 5.115 | 5.475 | 4.671 | 4.191 | 4.291 | 5.351 | 5.172 | 5.715 |
| | 800 nm (OD) | 1.963 | 2.311 | 2.478 | 2.110 | 1.892 | 1.939 | 2.413 | 2.342 | 2.577 |
| | 600/800 Ratio | 2.22 | 2.21 | 2.21 | 2.21 | 2.22 | 2.21 | 2.22 | 2.21 | 2.22 |
| Without Overturn Mixing / After Overturn Mixing | Content Ratio (%) | 75.3% | 89.0% | 93.0% | 88.6% | 74.7% | 74.7% | 104.2% | 90.9% | 101.9% |

| | Examples | 10 | 11 | 12 | 13 | 14 | 15 |
|---|---|---|---|---|---|---|---|
| With Overturn Mixing | 600 nm (OD) | 5.886 | 5.820 | 5.647 | 5.738 | 5.695 | 5.685 |
| | 800 nm (OD) | 2.646 | 2.627 | 2.549 | 2.592 | 2.571 | 2.566 |
| | 600/800 Ratio | 2.22 | 2.22 | 2.22 | 2.21 | 2.22 | 2.22 |
| Without Overturn Mixing | 600 nm (OD) | 3.548 | 4.331 | 5.034 | 5.082 | 5.377 | 5.103 |
| | 800 nm (OD) | 1.600 | 1.957 | 2.268 | 2.298 | 2.427 | 2.304 |
| | 600/800 Ratio | 2.22 | 2.21 | 2.22 | 2.21 | 2.22 | 2.21 |
| Without Overturn Mixing / After Overturn Mixing | Content Ratio (%) | 60.5% | 74.4% | 89.1% | 88.6% | 94.4% | 89.8% |

| | Examples | 16 | 17 | 18 | 19 | 20 | 21 |
|---|---|---|---|---|---|---|---|
| With Overturn Mixing | 600nm (OD) | 5.690 | 5.653 | 5.650 | 5.707 | 5.691 | 5.672 |
| | 800 nm (OD) | 2.578 | 2.555 | 2.548 | 2.578 | 2.565 | 2.563 |
| | 600/800 Ratio | 2.21 | 2.21 | 2.22 | 2.21 | 2.22 | 2.21 |
| Without Overturn Mixing | 600 nm (OD) | 4.422 | 3.593 | 4.593 | 4.752 | 4.654 | 4.271 |
| | 800 nm (OD) | 1.999 | 1.616 | 2.072 | 2.142 | 2.091 | 1.924 |
| | 600/800 Ratio | 2.21 | 2.22 | 2.22 | 2.22 | 2.23 | 2.22 |
| Without Overturn Mixing / After Overturn Mixing | Content Ratio (%) | 77.7% | 63.6% | 81.3% | 83.3% | 81.8% | 75.3% |

### (Results and Discussion)

The ratio of absorbance between 600 nm and 800 nm (600/800 ratio) represents the dispersibility of the particles in the solution. Using 2.21, i.e., the 600/800 ratio after overturn mixing of Comparative Example 1 (without additive), as a standard, if it was under the condition that the ratio of absorbance did not decrease by 0.10 or more, the dispersibility was evaluated to be good.

The content ratio (%) between (without overturn mixing) and (after overturn mixing) represents ease of particle sedimentation, and was evaluated as effective in inhibiting sedimentation of the additive if it was higher than that in Comparative Example 1 (without additive), and was evaluated as effective in inhibiting sedimentation than that with the conventional additive if it was higher than those in Comparative Examples 2 and 3 (sucrose added).

In Comparative Example 1, the 600/800 ratio after a lapse of 17 days was equal to "2.22" (without overturn mixing), against "2.21" (after overturn mixing), indicating no dispersibility problem. On the other hand, the content ratio between (without overturn mixing) and (after overturn mixing) after a lapse of 17 days was 54.6%, a low figure, and it was determined that the latex particles had sedimented. In Comparative Examples 2 and 3, the content ratios were 80.7% and 89.6%, which were higher than that of Comparative Example 1, and a certain inhibiting effect on latex sedimentation was recognized.

In Examples, first, the 600/800 ratios were, 2.21 to 2.22 in all conditions of Examples 1 to 21, indicating that a good dispersion state was maintained regardless of whether or not overturn mixing was used. Next, the content ratios (**%)** were higher values compared with that of Comparative Example 1 in all conditions of Examples 1 to 21, indicating a certain sedimentation inhibiting effect on the latex particles. In particular, in Example 3 (polyacrylic acid, molecular weight 25,000, final concentration 0.01**%**), Example 7 (polyacrylic acid, molecular weight 1,000,000, final concentration 0.01**%**), Example 8 (sodium polyacrylate, molecular weight 2,700 to 7,000, final concentration 0.01**%**), Example 9 (sodium polyacrylate, molecular weight 22,000 to 70,000, final concentration 0.01**%**), Example 14 (PEO-1, molecular weight 150,000 to 400,000, final concentration 0.01**%**), and Example 15 (PEO -3, molecular weight 600,000 to 1,100,000, final concentration 0.01**%**), the content ratios were exceeded the above content ratios (**%**) of Comparative Examples 2 and 3, in which the conventional additive was used, and it was found to have greater sedimentation inhibiting effects with respect to the latex particles than those in which the conventional additive was used.

### [Test Example 2: Absorbance Measurement in LTIA Method: Measurement of TARC]

The 2nd reagent of the LTIA measuring reagents was combined with the first reagent described below to perform measurements of TARC concentrations in TARC standard substance solutions and actual specimens, and to evaluate effects of additives contained in the 2nd reagent on detection sensitivity.

### [Comparative Example 1: Without Additive]

### 1. Measurement Method

### 1-1. LTIA Measuring Reagents

LTIA measuring reagents consisting of the 1st reagent and the 2nd reagent were prepared as shown below.

### (1) The 1st Reagent

100 mM MOPS-NaOH (pH 7.5)
500 mM NaCl
0.5% BSA

### (2) The 2nd Reagent

The same was used as in Comparative Example 1 of Test Example 1.

### 1-2. Samples

### TARC Calibration Substance

Sample 1: Saline Solution (TARC 0 pg/mL)
Sample 2: TARC 677 pg/mL
Sample 3: TARC 1,880 pg/mL
Sample 4: TARC 4,910 pg/mL
Sample 5: TARC 9,946 pg/mL
Sample 6: TARC 20,325 pg/mL

Arbitrary human serum specimens (Specimens 1 to 8) were used.

### 1-3. Measurement Procedure

The 1st and 2nd reagents were combined and the TARC concentration in a sample was measured using a Hitachi Automated Analyzer 3500. Specifically, 120 **µ**L of the 1st reagent was added to 2.4 **µ**L of the sample and kept at 37°C for 5 minutes, and then 40 **µ**L of the 2nd reagent was added and stirred. A change in absorbance accompanied by the formation of agglutination was then measured at a primary wavelength of 570 nm and a secondary wavelength of 800 nm over the next 5 minutes.

### 1-4. Analytical Method

A calibration curve was constructed from the concentrations and the changes in absorbance of the calibration substances, and the TARC concentration in the serum specimen was calculated.

With respect to the absorbance value under each measurement condition of the calibration substances, a relative sensitivity (each absorbance value divided by the absorbance value of Comparative Example 1 (%)) was calculated for the absorbance value of Comparative Example 1 (without additive).

### 2. Measurement Results

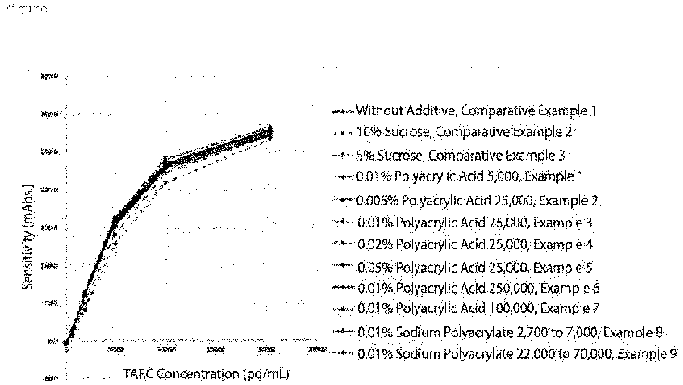

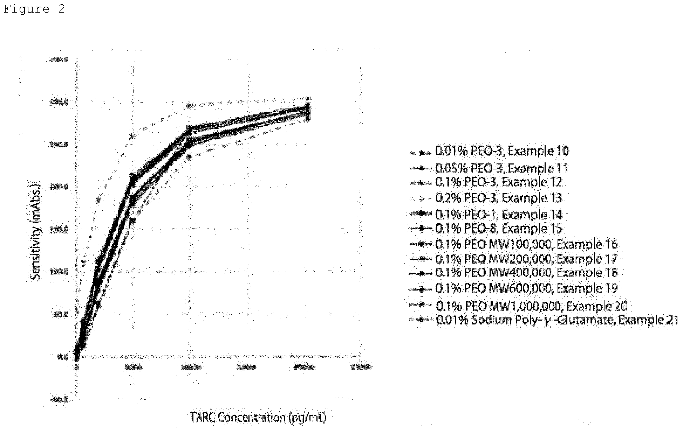

The measurement results for the calibration substances (Samples 1 to 6) are shown in Figures 1 and 2, and Table 3, and the relative sensitivities are shown in Table 4. The measurement results for the human serum specimens (Specimens 1 to 8) are also shown in Table 5.

### [Comparative Examples 2, 3: Formulated with Sucrose]

Measurements were performed in the same manner as in Comparative Example 1 of Test Examples 2, except that the same 2nd reagent was used as in Comparative Examples 2 and 3 of Test Examples 1. The measurement results for the calibration substances (Samples 1 to 6) are shown in Figures 1 and 2, and Table 3, and the relative sensitivities are shown in Table 4. The measurement results for the human serum specimens (Specimens 1 to 8) are also shown in Table 5.

### [Examples 1 to 21: Formulated with Additive]

Measurements were performed in the same manner as in Comparative Example 1 of Test Examples 2, except that the same 2nd reagent was used as in Examples 1 to 21 of Test Examples 1. The measurement results for the calibration substances (Samples 1 to 6) are shown in Figures 1 and 2, and Table 3, and the relative sensitivities are shown in Table 4. The measurement results for the human serum specimens (Specimens 1 to 8) are also shown in Table 5.

**[Table 3]**

| | | Absorbance (mAbs.) | | | | | | | | | | | |
|---|---|---|---|---|---|---|---|---|---|---|---|---|---|
| | Concentration (pg/mL) | Comparative Examples | | | Examples | | | | | | | | |
| | | 1 | 2 | 3 | 1 | 2 | 3 | 4 | 5 | 6 | 7 | 8 | 9 |
| Sample 1 | 0 | -3.0 | -3.6 | -2.5 | -1.9 | -2.6 | -2.0 | -1.5 | -2.0 | -2.1 | -2.2 | -2.4 | -2.1 |
| Sample 2 | 677 | 14.0 | 8.1 | 10.7 | 13.4 | 13.7 | 14.6 | 14.3 | 15.1 | 14.6 | 13.8 | 13.6 | 14.2 |
| Sample 3 | 1880 | 59.4 | 41.8 | 50.1 | 59.7 | 60.8 | 62.3 | 60.6 | 64.6 | 63.5 | 60.2 | 60.1 | 60.8 |
| Sample 4 | 4910 | 157.0 | 128.6 | 141.2 | 155.3 | 155.9 | 163.1 | 159.8 | 161.8 | 161.1 | 152.3 | 152.7 | 158.9 |
| Sample 5 | 9946 | 231.6 | 209.3 | 222.9 | 231.8 | 233.7 | 240.7 | 232.8 | 235.3 | 235.3 | 227.9 | 230.5 | 233.0 |
| Sample 6 | 20325 | 274.7 | 266.6 | 272.1 | 276.8 | 277.4 | 282.9 | 277.8 | 279.8 | 278.0 | 271.5 | 273.6 | 279.9 |

| | | Absorbance (mAbs.) | | | | | | | | | | | |
|---|---|---|---|---|---|---|---|---|---|---|---|---|---|
| | Concentration (pg/mL) | Examples | | | | | | | | | | | |
| | | 10 | 11 | 12 | 13 | 14 | 15 | 16 | 17 | 18 | 19 | 20 | 21 |
| Sample 1 | 0 | -2.4 | -0.4 | 6.6 | 53.4 | 1.7 | 9.4 | -0.9 | 0.6 | 4.7 | 4.0 | 6.0 | -2.2 |
| Sample 2 | 677 | 15.4 | 23.8 | 42.2 | 111.6 | 27.8 | 43.2 | 21.6 | 25.6 | 35.6 | 37.1 | 41.1 | 13.5 |
| Sample 3 | 1880 | 63.3 | 82.8 | 113.3 | 185.2 | 89.8 | 114.2 | 78.5 | 85.0 | 102.7 | 106.3 | 111.9 | 60.2 |
| Sample 4 | 4910 | 161.6 | 182.7 | 213.0 | 260.4 | 188.6 | 207.7 | 179.4 | 186.4 | 202.7 | 204.6 | 209.2 | 158.7 |
| Sample 5 | 9946 | 235.8 | 251.4 | 269.0 | 295.4 | 255.9 | 267.3 | 248.9 | 253.4 | 263.1 | 266.2 | 269.2 | 269.2 |
| Sample 6 | 20325 | 279.5 | 287.8 | 294.0 | 304.1 | 287.5 | 292.4 | 285.2 | 287.8 | 291.7 | 292.8 | 295.8 | 295.8 |

**[Table 4]**

| | | Absorbance (mAbs.) | | | | | | | | | | | |
|---|---|---|---|---|---|---|---|---|---|---|---|---|---|
| | Concentration (pg/mL) | Comparative Examples | | | Examples | | | | | | | | |
| | | 1 | 2 | 3 | 1 | 2 | 3 | 4 | 5 | 6 | 7 | 8 | 9 |
| Sample 2 | 677 | 100.0% | 68.2% | 77.1% | 89.7% | 95.6% | 97.6% | 92.9% | 100.6% | 97.9% | 93.8% | 93.5% | 95.6% |
| Sample 3 | 1880 | 100.0% | 72.6% | 84.1% | 98.6% | 101.4% | 103.0% | 99.5% | 106.7% | 105.0% | 99.8% | 100.0% | 100.7% |
| Sample 4 | 4910 | 100.0% | 82.6% | 89.8% | 98 .2% | 99.0% | 103.2% | 100.8% | 102.4% | 102.0% | 96.6% | 96.9% | 100.6% |
| Sample 5 | 9946 | 100.0% | 90.7% | 96.1% | 99.6% | 100.7% | 103.5% | 99.9% | 101.2% | 101.2% | 98.1% | 99.3% | 100.2% |
| Sample 6 | 20325 | 100.0% | 97.3% | 98.8% | 100.3% | 100.8% | 102.6% | 100.6% | 101.5% | 100.9% | 98.5% | 99.4% | 101.5% |

| | | Absorbance (mAbs.) | | | | | | | | | | | |
|---|---|---|---|---|---|---|---|---|---|---|---|---|---|
| | Concentration (pg/mL) | Examples | | | | | | | | | | | |
| | | 10 | 11 | 12 | 13 | 14 | 15 | 16 | 17 | 18 | 19 | 20 | 21 |
| Sample 2 | 677 | 104.7% | 142.1% | 209.4% | 342.1% | 153.8% | 198.8% | 132.1% | 147.4% | 181.5% | 194.4% | 206.8% | 92.4% |
| Sample 3 | 1880 | 105.2% | 133.3% | 171.0% | 211.1% | 141.3% | 167.9% | 127.1% | 135.3% | 157.0% | 163.9% | 169.8% | 100.0% |
| Sample 4 | 4910 | 102.5% | 114.4% | 129.0% | 129.4% | 116.9% | 123.9% | 112.7% | 116.2% | 123.8% | 125.4% | 127.0% | 100.6% |
| Sample 5 | 9946 | 101.5% | 107.4% | 111.9% | 103.2% | 108.4% | 109.9% | 106.5% | 107.8% | 110.1% | 111.8% | 112.2% | 115.7% |
| Sample 6 | 20325 | 101.5% | 103.8% | 103.5% | 90.3% | 102.9% | 101.9% | 103.0% | 103.4% | 103.3% | 104.0% | 104.4% | 107.3% |

**[Table 5]**

| | Measured Value (pg/mL) | | | | | | | | | | | |
|---|---|---|---|---|---|---|---|---|---|---|---|---|
| | Comparative Examples | | | Examples | | | | | | | | |
| | 1 | 2 | 3 | 1 | 2 | 3 | 4 | 5 | 6 | 7 | 8 | 9 |
| Specimen 1 | 295 | 268 | 253 | 270 | 274 | 241 | 264 | 342 | 278 | 352 | 358 | 268 |
| Specimen 2 | 360 | 395 | 349 | 347 | 368 | 369 | 385 | 369 | 392 | 389 | 408 | 353 |
| Specimen 3 | 475 | 357 | 386 | 398 | 492 | 448 | 476 | 489 | 565 | 563 | 569 | 592 |
| Specimen 4 | 793 | 829 | 838 | 879 | 820 | 862 | 782 | 820 | 747 | 846 | 779 | 881 |
| Specimen 5 | 18069 | 18019 | 17685 | 17798 | 16748 | 18269 | 17866 | 18252 | 18490 | 19829 | 18901 | 18968 |
| Specimen 6 | 10237 | 10194 | 9883 | 9903 | 10302 | 10112 | 10395 | 10306 | 10167 | 10834 | 10376 | 10781 |
| Specimen 7 | 5999 | 6127 | 6101 | 6159 | 6242 | 6249 | 6014 | 6199 | 6097 | 6538 | 6391 | 6258 |
| Specimen 8 | 12038 | 12461 | 11875 | 12319 | 12276 | 12280 | 12639 | 12518 | 12237 | 13218 | 12817 | 13591 |

| | Measured Value (pg/mL) | | | | | | | | | | | |
|---|---|---|---|---|---|---|---|---|---|---|---|---|
| | Examples | | | | | | | | | | | |
| | 10 | 11 | 12 | 13 | 14 | 15 | 16 | 17 | 18 | 19 | 20 | 21 |
| Specimen 1 | 319 | 327 | 269 | - | 348 | 320 | 339 | 317 | 332 | 347 | 359 | 299 |
| Specimen 2 | 395 | 411 | 360 | - | 426 | 388 | 388 | 377 | 380 | 405 | 443 | 379 |
| Specimen 3 | 446 | 506 | 515 | - | 557 | 490 | 532 | 478 | 498 | 500 | 525 | 470 |
| Specimen 4 | 814 | 877 | 894 | - | 923 | 846 | 850 | 853 | 905 | 820 | 904 | 847 |
| Specimen 5 | 18042 | 17727 | 18170 | - | 21650 | 19727 | 18254 | 18704 | 17057 | 17499 | 16509 | 17855 |
| Specimen 6 | 10301 | 10445 | 10484 | - | 10508 | 10145 | 10472 | 10550 | 10625 | 10330 | 10277 | 10405 |
| Specimen 7 | 6136 | 6249 | 6170 | - | 6630 | 6456 | 6268 | 6088 | 6220 | 6391 | 6395 | 6166 |
| Specimen 8 | 12451 | 12684 | 12778 | - | 12565 | 11716 | 12590 | 12639 | 12565 | 11937 | 12288 | 12813 |

### (Results and Discussion)

With respect to the measurement sensitivity in Table 4, preferable conditions are those in which the measurement sensitivity is equal to or greater than that of Comparative Example 1 without additive. However, for the reagents in Comparative Examples 2 and 3 with sucrose added, the decrease in sensitivity was more pronounced for samples with a lower TARC content, reaching 68.2% under the most reduced condition (Comparative Example 2 - Sample 2). Although a relative range of sensitivity decrease was narrowed with increasing the TARC content, it was found that the addition of sucrose which was the conventional additive had a negative effect on the reagent sensitivity.

By contrast, in the reagents containing polyacrylic acid 5,000, polyacrylic acid 25,000, polyacrylic acid 250,000, polyacrylic acid 100,000, sodium polyacrylate 2,700 to 7,000, sodium polyacrylate 22,000 to 70,000, or sodium poly-γ-glutamate, (Examples 1-9, 21), which are the components found out in the present application, the relative sensitivity was good, about 90% or more, regardless of the TARC content, and was comparable to the sensitivity of the reagent without additives. It was found that the reagents with any of these additives did not show any significant change in the measured values of human serum specimens, indicating that the TARC could be measured with high accuracy.

In addition, in the reagents containing PEO-1, PEO-3, PEO-8, and PEO Mv 100,000, 200,000, 400,000, 600,000, and 1,000,000, (Examples 10-20), the relative sensitivity was 100% or more, indicating a sensitizing effect. It was found that the reagents containing any of these additives did not show any significant change in the measured values of the human serum specimens, indicating that the TARC could be measured with high accuracy.

Among them, Example 14 (PEO-1, molecular weight 150,000 to 400,000, final concentration 0.01%) and Example 15 (PEO-3, molecular weight 600,000 to 1,100,000, final concentration 0.01%) showed a higher sedimentation inhibiting effect than those using previously known sedimentation inhibitors (Comparative Examples 2, 3), and was found to improve the measurement sensitivity, especially in samples with low concentrations of the target substance, indicating that it was an excellent component combining sedimentation inhibiting and sensitivity enhancing effects.

In summary, in the latex immunoagglutination measurement method, the addition of polyacrylic acid, sodium polyacrylate, sodium poly-**γ**-glutamate, or PEO did not decrease the sensitivity of the reagent and did not affect the performance of the reagent, and when any of them was added to the reagent, it was possible to measure target substances with high accuracy, indicating that they were useful as the latex sedimentation inhibitors of the present invention.

### [Industrial Applicability]

According to the latex particle dispersion liquid of the present invention, the sedimentation and/or agglutination of the latex particles is inhibited, and when applied to the latex immunoagglutination reagents, it becomes possible to perform measurement without doing redispersion by stirring or the like. In addition, the on-board stability of immunoagglutination reagents using the latex particles can be improved.

## Claims

1. A latex particle dispersion liquid comprising an aqueous medium as a dispersion medium, wherein the latex particle dispersion liquid comprises one or more compounds selected from the group consisting of polyacrylic acid, sodium polyacrylate, polyethylene oxide, and sodium poly-**γ**-glutamate.

2. The latex particle dispersion liquid according to claim 1, wherein the aqueous medium comprises water.

3. The latex particle dispersion liquid according to claim 1 or 2, wherein the latex particles are polymer particles derived from a monomer component containing a monomer having a styrene or vinylnaphthalene structure.

4. The latex particle dispersion liquid according to any one of claims 1 to 3, wherein the latex particles are latex particles carrying a substance capable of binding to a target substance.

5. The latex particle dispersion liquid according to claim 4, wherein the substance capable of binding to the target substance is an antigen or antibody to the target substance.

6. The latex particle dispersion liquid according to any one of claims 1 to 5, wherein the latex particle dispersion liquid is for use as an immunoagglutination reagent.

7. The latex particle dispersion liquid according to any one of claims 1 to 6, wherein an average particle diameter of the latex particles is 50 to 1,000 nm.

8. The latex particle dispersion liquid according to any one of claims 1 to 7, wherein a content of the latex particles is 0.01 to 0.10% by mass with respect to a total amount of the latex particle dispersion liquid.

9. A reagent kit for use in detecting a target substance in a specimen by an immunoagglutination method, wherein the reagent kit comprises the latex particle dispersion liquid according to any one of claims 1 to 8.

10. A method for detecting a target substance in a specimen by an immunoagglutination method, wherein the method comprises a step of bringing the specimen into contact with the latex particle dispersion liquid according to any one of claims 1 to 8.

11. A method for inhibiting sedimentation of latex particles carrying a substance capable of binding to a target substance in an immunoagglutination method, wherein the method comprises bringing the latex particles into contact with one or more compounds in an aqueous medium, wherein the compounds are selected from the group consisting of polyacrylic acid, sodium polyacrylate, polyethylene oxide, and sodium poly-γ-glutamate.
